# EUROPEAN PATENT APPLICATION

(11) **EP 2 832 729 A1**
(43) Date of publication of application: **04.02.2015**
(21) Application number: 13769121.8
(22) Date of filing: 29.03.2013
(51) Int. Cl.: C07D 263/32, C07B 61/00

(54) **N-PHENYL-N'-PHENYLSULFONYLPIPERAZINE DERIVATIVE AND METHOD FOR PRODUCING INTERMEDIATE FOR SAME**

(30) Priority: 29.03.2012 JP 2012076118
(71) Applicant: Shionogi & Co., Ltd., Osaka-shi, Osaka 541-0045 (JP)
(72) Inventor: KITAMURA, Hideyuki, Amagasaki-shi Hyogo 660-0813 (JP); GODA, Satoshi, Amagasaki-shi Hyogo 660-0813 (JP); YAMAMOTO, Mitsuru, Osaka-shi Osaka 5410045 (JP)
(74) Representative: Boult Wade Tennant
(86) International application number: PCT/JP2013/059459
(87) International publication number: WO 2013/147118

(57) **Abstract**

The present invention provides the salt and the crystal thereof of 2-(oxazole-2-yl)phenol derivative which are the intermediate in the process of producing N-phenyl-N'-phenylsulfonyl piperazine derivative, and the process of producing the salt as well as the process of producing N-phenyl-N'-phenylsulfonyl piperazine derivative through the salt.

A method of producing the salt and the crystal thereof of the compound represented by Formula(I): 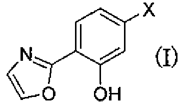 wherein X is halogen;
and the process of producing the salt as well as the process of producing N-phenyl-N'-phenylsulfonyl piperazine derivative through the salt.

## Description

### Technical Field

The present invention relates to a salt of a compound and a crystal thereof for useful in the process for producing N-phenyl-N'-phenylsulfonyl piperazine derivative. In addition, the present invention relates to a process for producing the salt of the compound and the process for producing N-phenyl-N'-phenylsulfonyl piperazine derivative through the salt.

### Background Art

N-phenyl-N'-phenylsulfonyl piperazine derivative (hereinafter, piperazine derivative) is known to exhibit various pharmaceutical activities. For example, Patent Document 1 discloses that a piperazine derivative has a TXA2 receptor antagonistic activity and has a platelet aggregation activity. Patent Document 2 discloses that a piperazine derivative has a TXA2 receptor antagonistic activity and an applicable disease for the derivative is thrombosis, myocardial infarction, arteriosclerosis, and hypertension. Patent Document 3 discloses that a piperazine derivative has a hydroxysteroid dehydrogenase inhibitory activity and an applicable disease for the derivative is type II diabetes mellitus and osteoporosis. Patent Document 4 discloses that a piperazine derivative has a CRTH2 receptor antagonistic activity, and an applicable disease for the derivative is allergic rhinitis and asthma. Patent Document 5 discloses that a piperazine derivative has a DP receptor antagonistic activity, and an applicable disease for the derivative is allergic rhinitis and asthma. Patent Document 6 discloses a process for producing a piperazine derivative. In these cited Document 1 to 6, only Patent Document 6 discloses 5-chloro-2-(1,3-oxazole-2-yl)phenol as a synthetic intermediate. However, it is not disclosed that the handling of 5-chloro-2-(1,3-oxazole-2-yl)phenol becomes difficult due to its sublimation in Patent Document 6.
[Patent Document 1]
   Publication of European Patent Application No.76996 specification
[Patent Document 2]
   Japanese Patent Application Laid-open Publication No.5-262751
[Patent Document 3]
   International Patent Publication No.2006/105127 pamphlet
[Patent Document 4]
   International Patent Publication No.2006/056752 pamphlet
[Patent Document 5]
   International Patent Publication No.2007/037187 pamphlet
[Patent Document 6]
   International Patent Publication No. 2008/123349 pamphlet

### Disclosure of the invention

### Problems to be solved by the Invention

As the result of extensive investigations about the efficient process for producing N-phenyl-N'-phenylsulfonyl piperazine derivative (hereinafter, piperazine derivative) for industrial process, the inventors of the present invention have found out that 5-chloro-2-(1,3-oxazole-2-yl)phenol which is used as an intermediate in Patent Document 6 is sublimable, and the inventors explored a intermediate which is not sublimable.

### Means for Solving the Problem

The inventors of the present invention found that a salt of 2-(1,3-oxazole-2-yl)phenol derivative which is useful as an intermediate in the process of producing the piperazine derivative and is not hygroscopic and sublimable, and the crystals thereof. In addition, the inventors of the present invention found that a process for producing the above intermediate and a process for producing the piperazine derivative via the above intermediate.

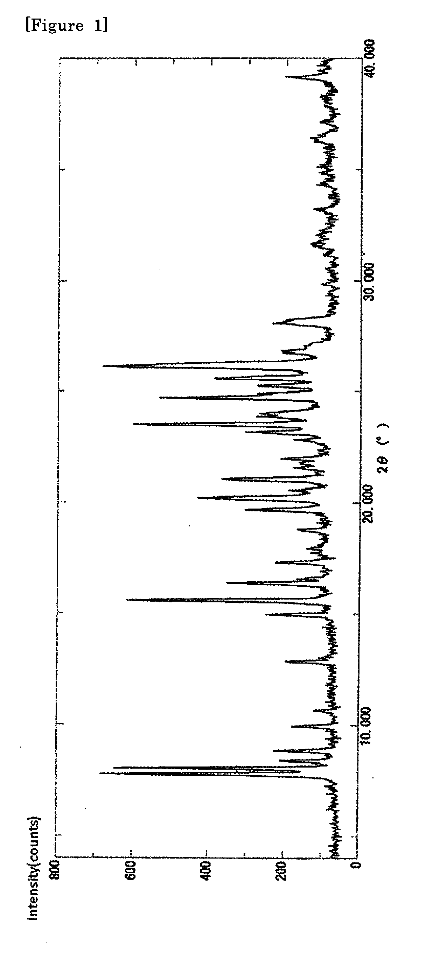

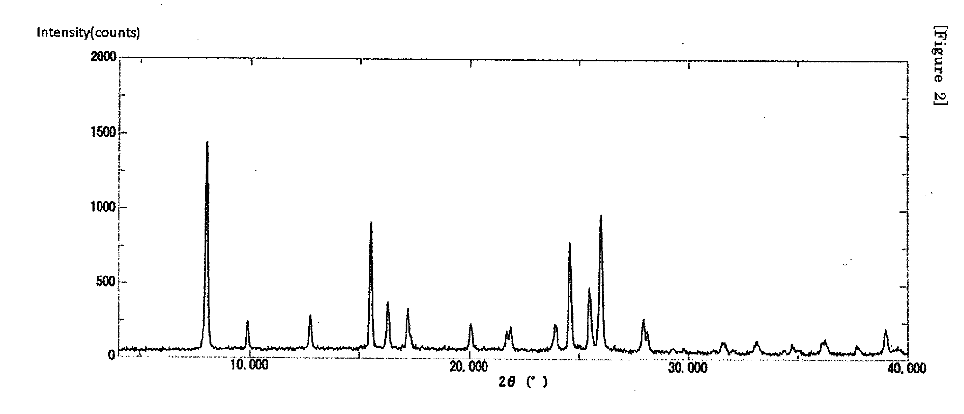

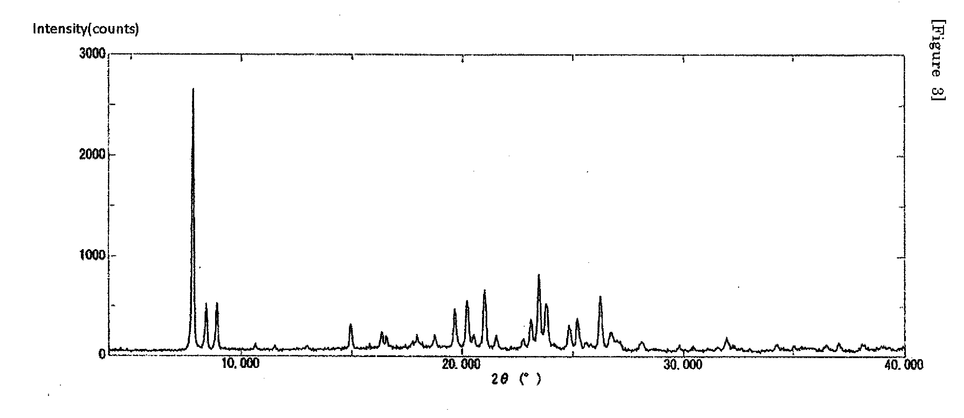

That is, the present invention relates to the following (1) to (10), (1A), (5A), (5B), (6A) to (6J) and (8A).
(1) A salt of the compound represented by Formula (I): 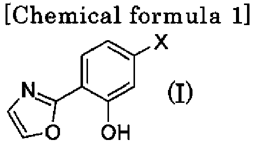 wherein X is halogen.
(1A) The salt according to (1), wherein said salt is a divalent salt.
(2) The salt according to (1), wherein said salt is magnesium salt or alkaline earth metal salt.
(3) The salt according to (1) or (2), wherein said salt is magnesium salt or calcium salt.
(4) The salt according to any one of (1) to (3) and (1A), wherein X is chlorine.
(5) A crystal of the salt of the compound represented by Formula (I): 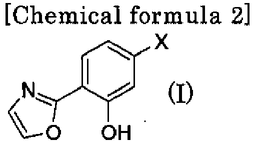 wherein X is halogen.
(5A) A crystal of a divalent salt of the compound represented by Formula (I): 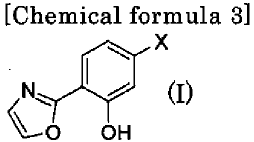 wherein X is halogen.
(5B) A crystal of magnesium salt or alkaline earth metal salt of the compound represented by Formula (I): 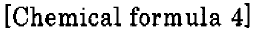 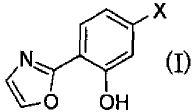 wherein X is halogen.
(6) The crystal according to (5), wherein said crystal is magnesium salt of the compound represented by Formula (I), when X is chlorine.
(6A) The crystal according to (6), wherein the crystal has peaks in diffraction angles (2θ) of : 7.8° ± 0.2°, 8.1° ± 0.2°, 8.4° ± 0.2°, 8.9° ± 0.2° and 15.6° ± 0.2° in an X-ray powder diffraction spectrum.
(6B) The crystal according to (6), wherein the crystal has peaks in diffraction angles (2θ) of : 7.8° ± 0.2°, 8.1° ± 0.2°, 8.4° ± 0.2°, 8.9° ± 0.2°, 15.6° ± 0.2°, 21.0° ± 0.2°, 23.5° ± 0.2°, 24.7° ± 0.2°, 25.6° ± 0.2° and 26.1° ± 0.2° in an X-ray powder diffraction spectrum.
(6C) The crystal according to (6), wherein the crystal has peaks in diffraction angles (2θ) of : 8.0° ± 0.2°, 15.5° ± 0.2°, 24.6° ± 0.2°, 25.5° ± 0.2° and 26.0° ± 0.2° in an X-ray powder diffraction spectrum.
(6D) The crystal according to (6), wherein the crystal has peaks in diffraction angles (2θ) of : 8.0° ± 0.2°, 9.9° ± 0.2°, 12.8° ± 0.2°, 15.5° ± 0.2°, 16.3° ± 0.2°, 17.2° ± 0.2°, 20.0° ± 0.2°, 24.6° ± 0.2°, 25.5° ± 0.2° and 26.0° ± 0.2° in an X-ray powder diffraction spectrum.
(6E) The crystal according to (6), wherein the crystal has peaks in diffraction angles (2θ) of : 7.8° ± 0.2°, 8.4° ± 0.2°, 8.8° ± 0.2°, 21.0° ± 0.2° and 23.4° ± 0.2° in an X-ray powder diffraction spectrum.
(6F) The crystal according to (6), wherein the crystal has peaks in diffraction angles (2θ) of: 7.8° ± 0.2°, 8.4° ± 0.2°, 8.8° ± 0.2°, 19.7° ± 0.2°, 20.2° ± 0.2°, 21.0° ± 0.2°, 23. 4° ± 0.2°, 23.8° ± 0.2°, 24.8° ± 0.2° and 26.2° ± 0.2° in an X-ray powder diffraction spectrum.
(6G) The crystal according to (6), wherein the crystal is characterized by an X-ray powder diffraction spectrum substantially identical with Figure 1.
(6H) The crystal according to (6), wherein the crystal is characterized by an X-ray powder diffraction spectrum substantially identical with Figure 2.
(61) The crystal according to (6), wherein the crystal is characterized by an X-ray powder diffraction spectrum substantially identical with Figure 3.
(6J) A process for producing the crystal according to any one of (5), (6), (5A), (5B) and (6A) to (6J).
(7) A process for producing magnesium salt or alkaline earth metal salt of the compound represented by Formula (I): 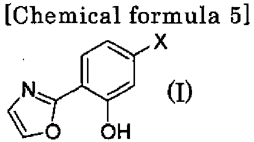 wherein X is as defined above;
   characterized by treating the compound represented by Formula (I) with magnesium halide, magnesium hydride, magnesium hydroxide, magnesium oxo acid salt, magnesium organic acid salt, alkaline earth metal halide, alkaline earth metal hydride, alkaline earth metal hydroxide, alkaline earth metal oxo acid salt or alkaline earth metal organic acid salt.
(7A) The process according to (7), comprising the step of producing the compound represented by Formula (I): 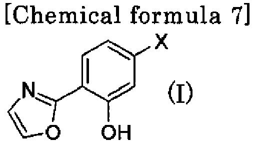 wherein X is halogen;
   by cyclization of the compound represented by Formula (II): 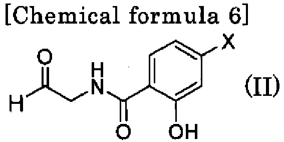 wherein X is as defined above; or a salt thereof.
(8) The process according to (7A), wherein the compound represented by Formula (II) or a salt thereof is cyclized in the presence of triphenylphosphine, a halogenating agent and a base.
(8A) The method according to (8), wherein said halogenating agent is a halogenating agent for triphenylphosphine.
(9) A process for producing the compound represented by Formula (VIII): 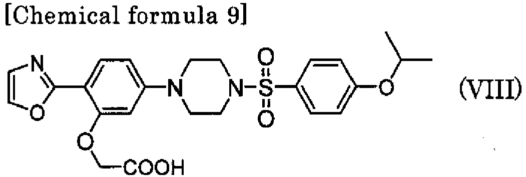 or a salt thereof, comprising the process through the salt of the compound represented by Formula (I): 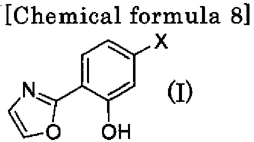
wherein X is halogen.
(9A) The process according to (9), wherein said salt of the compound represented by Formula (I): 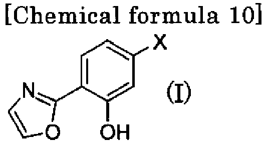 wherein X is halogen, is magnesium salt or alkaline earth metal salt.
(10) The process according to (9), wherein the compound represented by Formula (VIII): 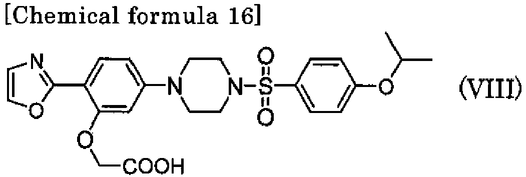 or a salt thereof;
   comprising treating magnesium salt or alkaline earth metal salt of the compound represented by Formula (I): 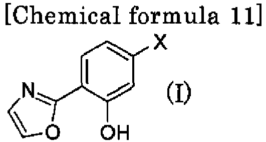 wherein X is halogen; with an acid to obtain the compound represented by Formula (I);
   the obtained compound represented by Formula (I) is reacted with the compound represented by Formula (IV): 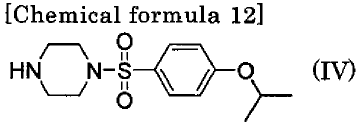 or a salt thereof, in the presence of a palladium catalyst, a phosphine ligand and a base to obtain the compound represented by Formula (V): 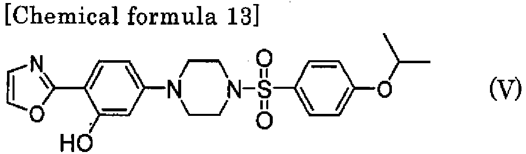 or a salt thereof;
   the obtained compound represented by Formula (V) or a salt thereof is reacted with the compound represented by Formula (VI): 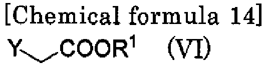 wherein Y is halogen, R¹ is alkyl; in the presence of a base to obtain the compound represented by Formula (VII): 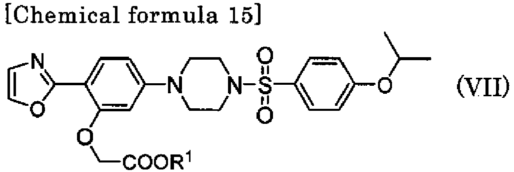 wherein R¹ is as defined above, or a salt thereof; and the obtained compound represented by Formula (VII) or a salt thereof is subjected to hydrolysis.

Terms used in this description are explained below. Each term, unless otherwise indicated, has the same meaning when it is used alone or together with other terms.

The term "halogen" includes fluorine, chlorine, bromine and iodine. For example, chlorine, bromine and iodine are exemplified. For example, chlorine and bromine are exemplified. For example, chlorine is exemplified.

The term "alkyl" includes a straight or branched chain monovalent hydrocarbon group of a carbon number of 1 to 8, as one embodiment a carbon number of 1 to 6, and as another embodiment a carbon number of 1 to 4. Examples include methyl, ethyl, n-propyl, isopropyl, n-butyl, isobutyl, sec-butyl, tert-butyl, n-pentyl, isopentyl, neo-pentyl, n-hexyl, isohexyl, n-heptyl, n-octyl, and the like.

Examples of the salt of the compound represented by Formula (I) include the salt of the compound represented by Formula (I) with alkaline metal (e.g., lithium, sodium, potassium or the like), alkaline earth metal (e.g., calcium, strontium, barium and radium), beryllium, magnesium, transition metal (e.g., zinc, iron or the like).

Examples of "divalent salt" include beryllium salt, magnesium salt, alkaline earth metal salt (e.g.,calcium salt, strontium salt, barium salt, and radium salt), transition metal salt (e.g., zinc salt, iron salt or the like). Preferably, magnesium salt or alkaline earth metal salt is exemplified.

Examples of "alkaline earth metal salt" include calcium salt, strontium salt, barium salt, and radium salt. Preferably, calcium salt is exemplified.

Examples of "magnesium halide, magnesium hydride, magnesium hydroxide, magnesium oxo acid salt, magnesium organic acid salt, alkaline earth metal halide, alkaline earth metal hydride, alkaline earth metal hydroxide, alkaline earth metal oxo acid salt or alkaline earth metal organic acid salt" include compounds comprising magnesium or alkaline earth metal (e.g., calcium, strontium, barium, and radium) with the above "halogen", hydrogen, hydroxy, oxo acid (e.g., carbonic acid, nitric acid, sulfuric acid, phosphoric acid, and the like) or organic acid (e.g., acetic acid, citric acid, glutamic acid, benzoic acid, stearic acid, and the like). For example, magnesium or alkaline earth metal halide such as magnesium fluoride, magnesium chloride, magnesium bromide, magnesium iodide, calcium fluoride, calcium chloride, calcium bromide, calcium iodide and the like;
magnesium or alkaline earth metal hydride such as magnesium hydride, calcium hydride and the like;
magnesium or alkaline earth metal hydroxide such as magnesium hydroxide, calcium hydroxide and the like;
magnesium or alkaline earth metal oxo acid salt such as magnesium carbonate, magnesium nitrate, magnesium sulfate, calcium carbonate, calcium nitrate, calcium sulfate and the like;
magnesium or alkaline earth metal organic acid salt such as magnesium acetate, magnesium citrate, magnesium glutamate, magnesium benzoate, magnesium stearate, calcium acetate, calcium citrate, calcium glutamate, calcium benzoate, calcium stearate and the like; are exemplified. For example, magnesium or alkaline earth metal halide is exemplified. For example, magnesium chloride and calcium chloride are exemplified. For example, magnesium chloride is exemplified.

The term "halogenating agent" includes a reagent to use for a reaction introducing halogen atom. For example, chlorinating agent such as chlorine, hexachloroethane and the like, brominating agent such as bromine, hexabromoethane and the like, and iodinating agent such as iodine are exemplified. For example, chlorinating agent such as chlorine, hexachloroethane and the like is exemplified. For example, chlorine is exemplified.

As the "base" in the above (8), organic base (e.g., trimethylamine, triethylamine, diisopropylethylamine, dicyclohexylamine, ethanolamine, diethanolamine, triethanolamine, meglumine, ethylenediamine, pyridine, picoline, quinoline, 1,8-diazabicyclo[5.4.0]undeca-7-ene, and the like), and the like can be used for the reaction. For example, trimethylamine is exemplified.

In the reaction to obtain the compound represented by Formula (V) or a salt thereof in the above (10), sodium tert-butoxide, sodium tert-pentoxide, cesium carbonate, sodium carbonate, potassium carbonate and the aqueous solution thereof can be used as the "base".

In the reaction to obtain the compound represented by Formula (VII) or a salt thereof in the above (10), sodium carbonate, potassium carbonate, cesium carbonate, sodium hydroxide, potassium hydroxide, cesium hydroxide and the like can be used as the "base". For example, potassium carbonate is exemplified.

In the above (10), inorganic acid (e.g., hydrochloric acid, sulfuric acid, nitric acid, carbonic acid, hydrobromic acid, phosphoric acid, hydroiodic acid and the like) and organic acid (e.g., formic acid, acetic acid, propionic acid, trifluoroacetic acid, citric acid, lactic acid, tartaric acid, oxalic acid, maleic acid, fumaric acid, mandelic acid, glutaric acid, malic acid, benzoic acid, phthalic acid, ascorbic acid, benzenesulfonic acid, p-toluenesulfonic acid, methanesulfonic acid, ethanesulfonic acid and the like) can be used as the "acid". For example, acetic acid is exemplified.

In the above (10), palladium acetate, palladium chloride, tris(dibenzylideneacetone)dipalladium, bis(dibenzylideneacetone)palladium, tetrakis(triphenylphosphine)palladium, [1,1'-bis(diphenylphosphino)ferrocene] dichloropalladium, dichlorobis(tri-o-tolylphosphine)palladium, dichlorobis(triphenylphosphine)palladium, palladium acetylacetonate, palladium carbon, dichlorobis(acetonitrile)palladium, bis(benzonitrile)palladium chloride, (1,3-diisopropylimidazol-2-ylidene)(3-chloropyridyl) palladium dichloride can be used as the "palladium catalyst. For example, palladium acetate is exemplified.

As "phosphine ligand", triphenylphosphine, tri(o-tolyl)phosphine, 9,9-dimethyl-4,5-bis(diphenylphosphino)xanthene, tri(tert-butyl)phosphine, di(tert-butyl)methyl phosphine, diadamantylbutylphosphine, 1,1'-bis(diphenylphosphino)ferrocene, 1,2-bis(diphenylphosphino)ethane, 1,3-bis(diphenylphosphino)propane, 1,4-bis(diphenylphosphino)butane, 2,2'-bis(diphenylphosphino)-1,1'-binaphthyl, tricyclohexylphosphine, diphenyl(cyclohexyl)phosphine, dicyclohexylphenylphosphine, diphenylphosphinoferrocene, 2-(di-tert-butylphosphino)biphenyl, tri(tert-butyl)phosphonium tetraphenylborate, di(tert-butyl)methyl phosphonium tetraphenylborate and the like can be used. For example, tri(tert-butyl)phosphonium tetraphenylborate is exemplified.

Additionally, in the above (10), bis(tri-tert-butyl phosphine)palladium, dichlorobis(triphenylphosphine)palladium, dichlorobis(tricyclohexylphosphine)palladium, dichlorobis(tri-o-tolylphosphine)palladium and the like, which is a complex of the above "palladium catalyst" and the above "phosphine ligand", can be used.

In the above (10), sodium carbonate, potassium carbonate, cesium carbonate, sodium hydroxide, potassium hydroxide, lithium hydroxide and the aqueous solution thereof can be used as a reagent for "hydrolysis". For example, aqueous sodium hydroxide solution is exemplified.

In the above (7A) and (8), "cyclization" includes the formation of a ring by reacting the side chain according to the following scheme. That is, "cyclization" includes the reaction to obtain the compound represented by Formula (I) by the steps wherein the compound represented by Formula (II) or a salt thereof is reacted with dihalogenated triphenylphosphorane which is derived from the reaction of triphenylphosphine and halogenating agent as defined above "halogenating agent", to obtain the compound represented by Formula (I-a) or a salt thereof in the step "a", and the obtained compound or a salt thereof in the step "a" is treated with the base as defined "base" in above (8) in the step "b". 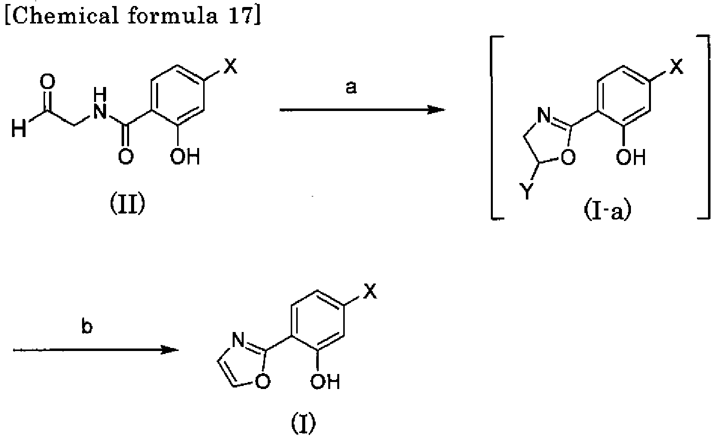 wherein X is halogen, Y is halogen derived from dihalogenated triphenylphosphorane.

The compounds represented by Formula (I) are not limited to specific isomers but include all possible isomers (e.g., keto-enol isomers, imine-enamine isomers, diastereoisomers, enantiomers, rotamers or the like), racemates or mixtures thereof.

One or more hydrogen, carbon and/or other atoms in the compounds of Formula (I), and the like may be replaced with isotopes of hydrogen, carbon and/or other atoms respectively. Examples of isotopes include hydrogen, carbon, nitrogen, oxygen, phosphorus, sulfur, fluorine, iodine and chlorine, such as ²H, ³H, ¹¹C, ¹³C, ¹⁴C, ¹⁵N, ¹⁸O, ¹⁷O, ³¹P, ³²P, ³⁵S, ¹⁸F, ¹²³I and ³⁶Cl respectively. The compound represented by Formula (I) includes the compounds replaced with these isotopes. The compounds replaced with the above isotopes are useful as medicines and include all of radiolabeled compounds of the compound represented by Formula (I). A "method of radiolabeling" in the manufacture of the "radiolabeled compounds" is encompassed by the present invention, and the "radiolabeled compounds" are useful for studies on metabolized drug pharmacokinetics, studies on binding assay and/or diagnostic tools.

A radiolabeled compound of the compound represented by Formula (I) can be prepared using well-known methods in this field of the invention. For example, a tritium-labeled compound represented by Formula (I) can be prepared by introducing a tritium to a certain compound represented by Formula (I), through a catalytic dehalogenation reaction using a tritium. This method comprises reacting with an appropriately-halogenated precursor of the compound of Formula (I) with tritium gas in the presence of an appropriate catalyst, such as Pd/C, and in the presence or absent of a base. The other appropriate method of preparing a tritium-labeled compound can be referred to "Isotopes in the Physical and Biomedical Sciences, Vol. 1, Labeled Compounds (Part A), Chapter 6 (1987)". A ¹⁴C-labeled compound can be prepared by using a starting material having ¹⁴C.

The compounds used in this specification can be used appropriately as a salt with an acid or a salt with a base according to the compounds. For example, salts with a base such as salts with alkaline metal (e.g., lithium, sodium, potassium or the like), alkaline earth metal (e.g., calcium, strontium, barium and radium), beryllium, magnesium, transition metal (e.g., zinc, iron or the like), ammonia, organic bases (e.g., trimethylamine, triethylamine, dicyclohexylamine, ethanolamine, diethanolamine, triethanolamine, meglumine, diethanolamine, ethylenediamine, pyridine, picoline, quinoline or the like) or amino acids; or salts with an acid such as salts with inorganic acids (e.g., hydrochloric acid, sulfuric acid, nitric acid, carbonic acid, hydrobromic acid, phosphoric acid, hydroiodic acid or the like) or organic acids (e.g., formic acid, acetic acid, propionic acid, trifluoroacetic acid, citric acid, lactic acid, tartaric acid, oxalic acid, maleic acid, fumaric acid, mandelic acid, glutaric acid, malic acid, benzoic acid, phthalic acid, ascorbic acid, benzenesulfonic acid, p-toluenesulfonic acid, methanesulfonic acid, ethanesulfonic acid or the like) are exemplified.

For example, salts with alkaline metal (e.g., lithium, sodium, potassium or the like), alkaline earth metal (e.g., calcium, barium or the like), magnesium, transition metal (e.g., zinc, iron or the like) are exemplified.

For example, salts with alkaline metal (e.g., lithium, sodium, potassium or the like), alkaline earth metal (e.g., calcium, barium or the like), magnesium are exemplified.

For example, salts with alkaline earth metal (e.g., calcium, barium or the like), magnesium are exemplified.

For example, salts with hydrochloric acid, sulfuric acid, phosphoric acid, tartaric acid, methanesulfonic acid are exemplified.

These salts can be formed in accordance with the conventional method.

The compound of Formula (I) and the like, or a salt thereof in the present invention may form solvate (e.g., hydrate or the like) and/or crystal polymorphism. The present invention encompasses those various solvate and crystal polymorphism. "Solvates" may be those wherein any numbers of solvent molecules (e.g., water molecules or the like) are coordinated with the compound of Formula (I) and the like. When the compound of Formula (I) and the like, or a salt thereof are allowed to stand in the atmosphere, the compound may absorb water, resulting in attachment of adsorbed water or formation of hydrate. In addition, recrystallization of the compound of Formula (I) and the like, or a salt thereof may produce crystal polymorphism.

The salt of the compound represented by Formula (I), for example, magnesium salt or calcium salt, especially, magnesium salt doesn't indicate the result of no sublimation and/or low hygroscopicity in the evaluation of sublimation and/or hygroscopicity test described in Test Examples 1 to 3. These results indicate excellent effect so that intermediate can be stably stored in the long period when performing a large scale synthesis as industrial production of medicaments.

### Effect of the invention

The salt of the compound and crystal thereof of the present invention are useful for the industrial production of N-phenyl-N'-phenylsulfonylpiperazine derivative, because they don't have the sublimation.

### Best mode for carrying out the invention

### Process A

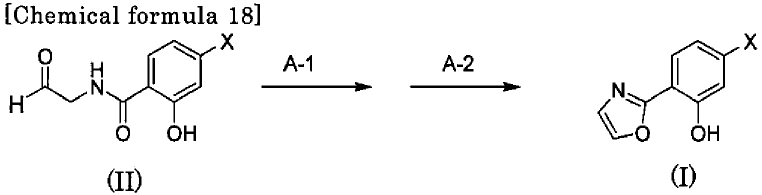

wherein X is halogen.

Process A is a process for producing magnesium salt or alkaline earth metal salt of the compound represented by Formula (I) and crystal thereof by Step A-1 and Step A-2.

Step A-1: The compound represented by Formula (II) or a salt thereof is cyclized to obtain the compound represented by Formula (I).

Step A-2: The obtained compound in Step A-1 is treated with magnesium halide, magnesium hydride, magnesium hydroxide, magnesium oxo acid salt, magnesium organic acid salt, alkaline earth metal halide, alkaline earth metal hydride, alkaline earth metal hydroxide, alkaline earth metal oxo acid salt or alkaline earth metal organic acid salt.

The compound represented by Formula (II) or a salt thereof may be cyclized in the presence of triphenylphosphine, halogenating agent and a base in Step A-1.

In addition, the compound represented by Formula (II) or a salt thereof may be cyclized by the reaction with a solution prepared by reacting triphenylphosphine and halogenating agent, followed by treatment with a base.

Triphenylphosphine may be used in an amount of 1 to 5 molar equivalent(s) relative to the compound represented by Formula (II) or a salt thereof.

Halogenating agent may be used in an amount of 1 to 5 molar equivalent(s) relative to the compound represented by Formula (II) or a salt thereof.

As the base, organic base (e.g., trimethylamine, triethylamine, diisopropylethylamine, dicyclohexylamine, ethanolamine, diethanolamine, triethanolamine, meglumine, ethylenediamine, pyridine, picoline, quinoline, 1,8-diazabicyclo[5.4.0]undeca-7-ene, or the like) may be used. For example, trimethylamine may be used. It may be used in an amount of 2 to 10 molar equivalents relative to the compound represented by Formula (II) or a salt thereof.

In Step A-2, magnesium halide, magnesium hydride, magnesium hydroxide, magnesium oxo acid salt, magnesium organic acid salt, alkaline earth metal halide, alkaline earth metal hydride, alkaline earth metal hydroxide, alkaline earth metal oxo acid salt or alkaline earth metal organic acid salt may be used in an amount of 0.5 to 5 molar equivalent(s) relative to the compound represented by Formula (II) or a salt thereof.

The reaction temperature may be room temperature to the solvent reflux temperature.

The reaction time may be 30 minutes to 24 hours.

As the solvent, dioxane, tetrahydrofuran, N,N -dimethylformamide, or acetonitrile may be used alone or in combination. For example, acetonitrile may be used.

Meanwhile, the obtained compound may be isolated and purified by using the generally used method such as silica gel chromatography, recrystallization, or/and distillation. Also, it may be used in the next reaction without being purified.

X-ray diffraction patterns of magnesium salt or alkaline earth metal salt of the compound represented by Formula (I) generated in the production method can be obtained by X-ray powder diffraction.

### Process B

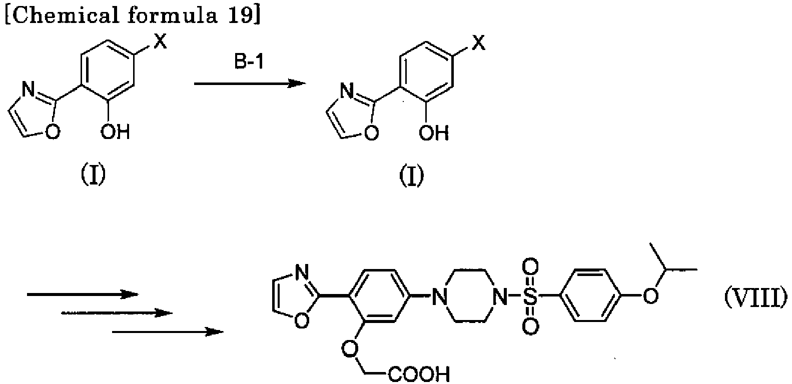

wherein X is halogen.

Process B is a process for producing the compound represented by Formula (VIII) or a salt thereof, by treating magnesium salt or alkaline earth metal salt of the compound represented by Formula (I) with an acid to obtain the compound represented by Formula (I) in Step B-1. Herein, the compound represented by Formula (VIII) or a salt thereof can be prepared in accordance with the method described in Patent Document 6.

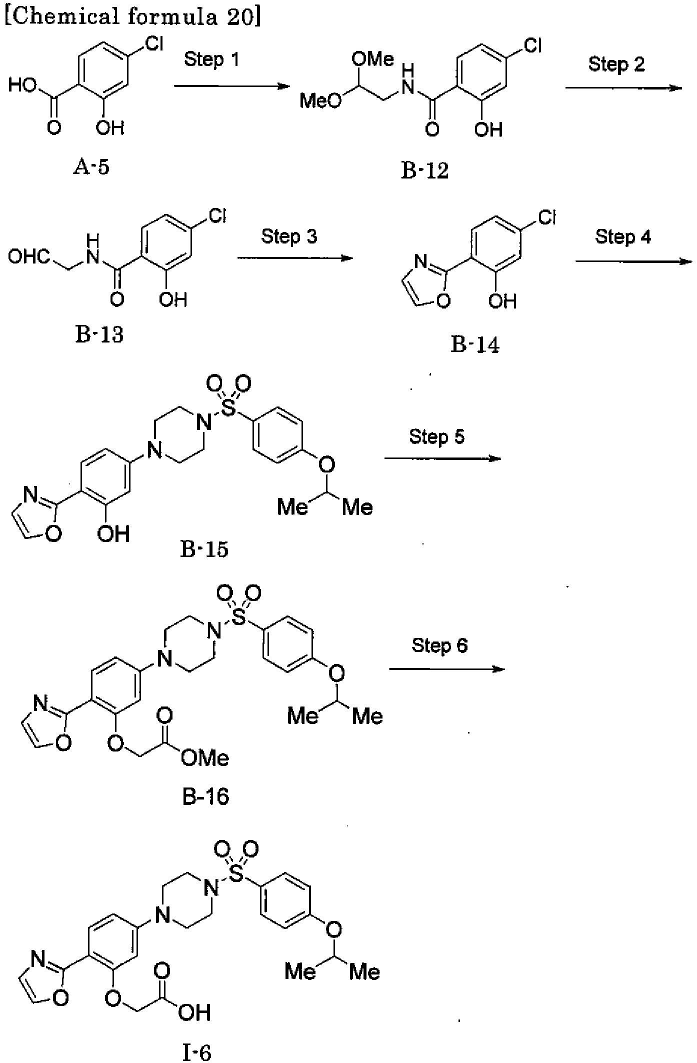

In Step B-1, inorganic acid (e.g., hydrochloric acid, sulfuric acid, nitric acid, carbonic acid, hydrobromic acid, phosphoric acid, hydroiodic acid and the like) and organic acid (e.g., formic acid, acetic acid, propionic acid, trifluoroacetic acid, citric acid, lactic acid, tartaric acid, oxalic acid, maleic acid, fumaric acid, mandelic acid, glutaric acid, malic acid, benzoic acid, phthalic acid, ascorbic acid, benzenesulfonic acid, p-toluenesulfonic acid, methanesulfonic acid, ethanesulfonic acid and the like) may be used as the acid. For example, acetic acid is exemplified. It may be used in an amount of 0.5 to 15 molar equivalent(s) relative to the magnesium salt of the compound represented by Formula (I) or alkaline earth metal salt of the compound represented by Formula (I).

The reaction temperature may be room temperature to the solvent reflux temperature.

The reaction time may be 30 minutes to 24 hours.

As the solvent, dioxane, benzene, toluene, hexane, tetrahydrofuran, N,N-dimethylformamide, acetonitrile, methanol, ethanol, propanol, buthanol, water and acetic acid may be used alone or in combination. For example, the mixture of toluene, methanol and water may be used.

Meanwhile, the obtained compound may be isolated and purified by using the generally used method such as silica gel chromatography, recrystallization, or/and distillation. Also, it may be used in the next reaction without being purified.

### X-Ray Powder Diffraction (XRPD)

Crystalline organic compounds consist of a large number of atoms that are arranged in a periodic array in three-dimensional space. The structural periodicity normally manifests distinct physical properties, such as sharp, explicit spectral features by most spectroscopic probes (e.g., X-ray diffraction, infrared and solid state NMR). X-ray powder diffraction (XRPD) is acknowledged to be one of the most sensitive methods to determine the crystallinity of solids. Crystals yield explicit diffraction maxima that arise at specific angles consistent with the lattice interplanar spacings, as predicted by Bragg's law. On the contrary, amorphous materials do not possess long-range order. They often retain additional volume between molecules, as in the liquid state. Amorphous solids normally unveil a featureless XRPD pattern with broad, diffuse halos because of the absence of the long range order of repeating crystal lattice.

A crystalline form disclosed in this description of magnesium bis[5-chloro-2-(1,3-oxazole-2-yl)phenolate](I-2) preferably has a distinguishable X-ray powder diffraction profile. For example, a crystal comprising magnesium bis[5-chloro-2-(1,3-oxazole-2-yl)phenolate](I-2) can be preferably distinguished by the presence of characteristic diffraction peaks. The characteristic diffraction peaks used in this description are the ones selected from the observed diffraction peaks. They are preferably 20, more preferably 10, and most preferably 5 peaks in a diffraction pattern.

Since an error in the range of ±0.2° may occur in diffraction angles (2θ) in X-ray powder diffraction, in general, the value of the above diffraction angle should be understood as including values in a range of around ±0.2°. Therefore, the present invention includes not only crystals whose diffraction angles of the peaks in X ray powder diffraction perfectly match, bust also crystals whose diffraction angles of the peaks match within an error of around ±0.2°.

In general, it is known that the relative intensities of various peaks shown in the Tables and Figures below may vary due to a number of factors such as orientation effects of crystals in the X-ray beam or the purity of the material being analyzed or the degree of crystallinity of the sample. The peak positions may also shift for variations in sample height. Furthermore, measurements using a different wavelength will result in different shifts according to the Bragg equation (nλ=2d sin θ). Such further XPRD patterns obtained by using a different wavelength are within the scope of the present invention.

TG/DTA is one of the major measuring methods of a thermal analysis, and is the method of measuring the weight and the thermal property of a substance as an aggregate of an atom and a molecule.

TG/DTA is the method of measuring change of the weight and the quantity of heat concerning the temperature or time of an active pharmaceutical ingredient, and TG(thermo gravity) and a DTA(Differential Thermal Analysis)curve are obtained by plotting the obtained data to temperature or time. From TG/DTA curve, the information on the weight about decomposition of an active pharmaceutical ingredient, dehydration, oxidation, reduction, sublimation, and evaporation and quantity of heat change can be acquired.

In TG/DTA, a "melting point" means onset temperature.

It is known that the temperature and the weight change observed can be dependent on heating rate, the sample preparation technique to be used, and a specific device about TG/DTA. In authorization of the identity of crystal, an overall pattern is important and may change with measurement conditions to some degree.

Moisture adsorption-desorption isotherm is one of means for measuring the adsorption or desorption of moisture. It is a method of measuring the mass change as a function of the relative humidity at constant temperature, and under the conditions where adsorption or desorption is essentially occurring independently of time (equilibrium conditions). Moisture adsorption-desorption isotherm is obtained by plotting relative humidities versus the ratio(%) of the mass increase compared to the mass when the relative humidity of 0%. Accordingly, it is possible to obtain the information about hygroscopicity or dehumidifying nature of the sample.

The present invention is explained in more detail by examples below, but these examples do not limit the present invention.

The definition of abbreviations used in the examples is as follows, Me:methyl, DMSO:dimethylsulfoxide.

### [Example 1]

### Synthesis of magnesium bis[5-chloro-2-(1,3-oxazole-2-yl)phenolate](I-2) and calcium bis[5-chloro-2-(1,3-oxazole-2-yl)phenolate](I-7)

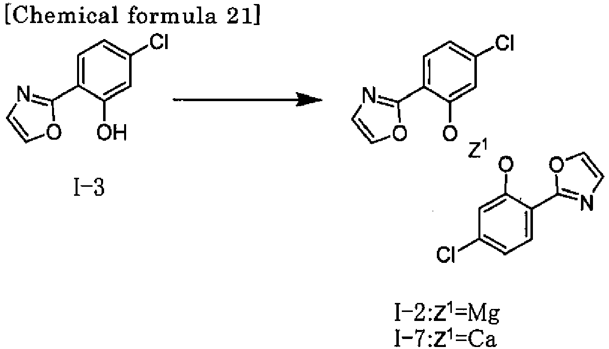

wherein Z¹ is magnesium or calcium.

### (1) Synthesis of magnesium bis[5-chloro-2-(1,3-oxazole-2-yl)phenolate](I-2)

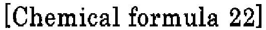

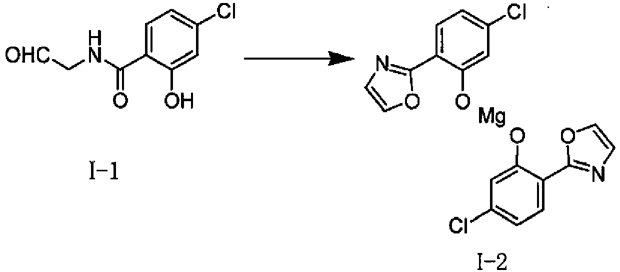

Triphenylphosphine(140.96 g, 537.41 mmol) was suspended in acetonitrile(550 mL). To the reaction mixture was added chlorine(37.79 g, 532.93 mmol) at 20°C to prepare dichlorotriphenylphosphorane in acetonitrile(611.28 g).

4-Chloro-2-hydroxy-N-(2-oxoethyl)benzamide(I-1, 5.97 g, 27.94 mmol) was suspended in acetonitrile(12mL). The above dichlorotriphenylphosphorane in acetonitrile(42.85 g) was added dropwise to the reaction mixture at 25°C over 1 hour 45 minutes. The reaction mixture was stirred for 55 minutes. To the reaction mixture was added dropwise triethylamine(11.31 g, 111.77 mol) at 25°C over 2 hours. The reaction mixture was stirred at 25°C for 2 hours 52 minutes. To the reaction mixture was added water(12mL) at 25°C, and the mixture was warmed up to 60°C. Magnesium chloride hexahydrate(2.84 g, 13.98 mmol) was dissolved in water(6mL) to give aqueous magnesium chloride solution. It was added dropwise to the reaction mixture at 60°C over 1 hour. The reaction mixture was cooled to 25°C, then stood overnight. The resulting crystals were collected by filtration to afford magnesium bis[5-chloro-2-(1,3-oxazole-2-yl)phenolate] (I-2, 5.08 g, 96.1%).
Elemental analysis: C 51.67%, H 2.60%, Cl 16.76%, N 6.72%, Mg 5.65%
¹H-NMR (DMSO-d6) δppm: 6.33(dd, J=8.4 Hz, 2.1 Hz, 1H), 6.63(d, J=2.1 Hz, 1H), 6.88(d, J=0.9 Hz, 1H), 7.56(dd, J=15.9 Hz, 7.5 Hz, 1H), 7.86(d, J=0.9 Hz, 1H)
Result of X-ray powder diffraction is shown in Table 1 and Table 2, and Figure 1.
Diffraction angles of major peaks: 2θ=7.8, 8.1, 8.4, 8.9, 15.6

**[Table 1]**

| Angle 2-Theta ° | d value Ansgstrom | Intensity % |
|---|---|---|
| 7.80 | 11.33 | 100 |
| 8.06 | 10.96 | 100 |
| 8.40 | 10.52 | 23 |
| 8.86 | 9.97 | 28 |
| 9.96 | 8.87 | 18 |
| 10.64 | 8.31 | 11 |
| 12.84 | 6.89 | 23 |
| 14.94 | 5.93 | 26 |
| 15.58 | 5.68 | 90 |
| 16.36 | 5.41 | 45 |
| 16.56 | 5.35 | 18 |
| 17.30 | 5.12 | 28 |
| 17.94 | 4.94 | 11 |
| 18.78 | 4.72 | 12 |
| 19.10 | 4.64 | 9 |
| 19.66 | 4.51 | 39 |
| 20.20 | 4.39 | 56 |
| 21.02 | 4.22 | 45 |
| 21.54 | 4.12 | 11 |
| 21.78 | 4.08 | 10 |
| 21.96 | 4.04 | 22 |

**[Table 2]**

| Angle 2-Theta ° | d value Ansgstrom | Intensity % |
|---|---|---|
| 22.80 | 3.90 | 11 |
| 23.14 | 3.84 | 34 |
| 23.48 | 3.79 | 85 |
| 23.86 | 3.73 | 27 |
| 24.00 | 3.70 | 26 |
| 24.68 | 3.60 | 74 |
| 24.88 | 3.58 | 26 |
| 25.24 | 3.53 | 27 |
| 25.56 | 3.48 | 44 |
| 26.10 | 3.41 | 95 |
| 26.72 | 3.33 | 20 |
| 26.84 | 3.32 | 19 |
| 27.00 | 3.30 | 10 |
| 28.08 | 3.18 | 26 |
| 28.24 | 3.16 | 21 |
| 31.48 | 2.84 | 10 |
| 31.62 | 2.83 | 10 |
| 31.94 | 2.80 | 8 |
| 36.38 | 2.47 | 13 |
| 39.14 | 2.30 | 22 |

The crystals obtained in accordance with the above method was confirmed the mixture of polymorphism by comparison to the result of X-ray powder diffraction of polymorphism of the following single substance.

The single substance of polymorphism was obtained in accordance with the following method.

To 5-chloro-2-(1,3-oxazole-2-yl)phenol (I-3, 5.00 g, 25.6 mmol) were added acetonitrile(70mL), water(14mL) and triethylamine(3.15 g, 31.1 mmol) at room temperature. The reaction mixture was warmed up to 60°C. Magnesium chloride hexahydrate (2.89 g, 14.2 mmol) was dissolved in water(5mL) to give aqueous magnesium chloride solution. It was added dropwise to the reaction mixture over 1 hour. The reaction mixture was cooled to room temperature, then stirred overnight. The resulting crystals were collected by filtration to afford magnesium bis[5-chloro-2-(1,3-oxazole-2-yl)phenolate] (1-2, 5.12 g, 96.9%) as crystal form I.

Result of X-ray powder diffraction is shown in Table 3 and Figure 2.

Diffraction angles of major peaks: 2θ=8.0, 15.5, 24.6, 25.5, 26.0

**[Table 3]**

| Angle 2-Theta ° | d value Ansgstrom | Intensity % |
|---|---|---|
| 7.86 | 11.24 | 11 |
| 7.94 | 11.13 | 44 |
| 8.00 | 11.04 | 100 |
| 8.06 | 10.96 | 38 |
| 9.88 | 8.95 | 15 |
| 12.76 | 6.93 | 17 |
| 15.48 | 5.72 | 67 |
| 16.26 | 5.45 | 26 |
| 16.34 | 5.42 | 13 |
| 17.14 | 5.17 | 17 |
| 17.20 | 5.15 | 20 |
| 20.04 | 4.43 | 15 |
| 21.84 | 4.07 | 12 |
| 21.88 | 4.06 | 12 |
| 23.88 | 3.72 | 15 |
| 23.92 | 3.72 | 13 |
| 24.56 | 3.62 | 61 |
| 24.62 | 3.61 | 31 |
| 24.68 | 3.60 | 12 |
| 25.46 | 3.50 | 34 |
| 25.88 | 3.44 | 17 |
| 25.96 | 3.43 | 54 |
| 26.00 | 3.42 | 72 |
| 27.92 | 3.19 | 19 |
| 27.96 | 3.19 | 17 |
| 28.12 | 3.17 | 13 |
| 39.00 | 2.31 | 15 |

The crystals of polymorphism mixture or crystal form I in acetone was stirred under refluxing for 4.5 hours, then the resulting crystals were collected by filtration to afford crystal form II.

Result of X-ray powder diffraction is shown in Table 4 and Table 5, and Figure 3.

Diffraction angles of major peaks: 2θ=7.8, 8.4, 8.8, 21.0, 23.4

**[Table 4]**

| Angle 2-Theta ° | d value Ansgstrom | Intensity % |
|---|---|---|
| 7.66 | 11.53 | 9 |
| 7.80 | 11.33 | 100 |
| 8.32 | 10.62 | 6 |
| 8.40 | 10.52 | 19 |
| 8.44 | 10.47 | 13 |
| 8.80 | 10.04 | 7 |
| 8.84 | 10.00 | 15 |
| 8.88 | 9.95 | 19 |
| 14.92 | 5.93 | 10 . |
| 15.00 | 5.90 | 7 |
| 16.34 | 5.42 | 8 |
| 16.54 | 5.36 | 7 |
| 17.94 | 4.94 | 7 |
| 19.60 | 4.53 | 9 |
| 19.66 | 4.51 | 16 |
| 19.70 | 4.50 | 11 |
| 20.16 | 4.40 | 14 |
| 20.20 | 4.39 | 20 |
| 20.24 | 4.38 | 15 |
| 20.96 | 4.23 | 17 |
| 21.00 | 4.23 | 24 |

**[Table 5]**

| Angle 2-Theta ° | d value Ansgstrom | Intensity % |
|---|---|---|
| 21.54 | 4.12 | 6 |
| 23.06 | 3.85 | 9 |
| 23.10 | 3.85 | 12 |
| 23.44 | 3.79 | 29 |
| 23.46 | 3.79 | 32 |
| 23.56 | 3.77 | 9 |
| 23.74 | 3.74 | 13 |
| 23.78 | 3.74 | 18 |
| 23.84 | 3.73 | 14 |
| 24.78 | 3.59 | 9 |
| 24.82 | 3.58 | 11 |
| 24.86 | 3.58 | 8 |
| 25.14 | 3.54 | 9 |
| 25.18 | 3.53 | 13 |
| 26.20 | 3.40 | 23 |
| 26.22 | 3.40 | 24 |
| 26.26 | 3.39 | 16 |
| 26.68 | 3.34 | 7 |
| 26.72 | 3.33 | 7 |
| 26.76 | 3.33 | 6 |
| 31.96 | 2.80 | 6 |

### (2) Synthesis of calcium bis[5-chloro-2-(1,3-oxazole-2-yl)phenolate](I-7)

To 5-chloro-2-(1,3-oxazole-2-yl)phenol (1-3, 1.00 g, 5.1 mmol) were added acetonitrile(14mL), water(2mL) and triethylamine(0.63 g, 6.1 mmol) at room temperature. The reaction mixture was warmed up to 60°C. Calcium chloride dihydrate (0.42 g, 2.8 mmol) was dissolved in water(3mL) to give aqueous calcium chloride solution. It was added dropwise to the reaction mixture over 1 hour. The reaction mixture was cooled to 25°C, then stirred for 1 hour 20 minutes. The resulting solids were collected by filtration to afford calcium bis[5-chloro-2-(1,3-oxazole-2-yl)phenolate] (1-7, 1.06 g, 96.2%).
Elemental analysis: C 49.31%, H 2.58%, Cl 16.39%, N 6.30%, Ca 8.79%
¹H-NMR (DMSO-d6) δppm: 6.27(d, J=6 Hz, 1H), 6.50(s 1H), 7.27(s, 1H), 7.58(d, J=6 Hz, 1H), 7.94(s, 1H)

### [Example 2]

### Synthesis of sodium 5-chloro-2-(1,3-oxazole-2-yl)phenolate (I-8), potassium 5-chloro-2-(1,3-oxazole-2-yl)phenolate(I-9) and lithium 5-chloro-2-(1,3-oxazole-2-yl)phenolate(I-10), which were used for hygroscopicity test of the salts of 5-chloro-2-(1, 3-oxazole-2-yl)phenol.

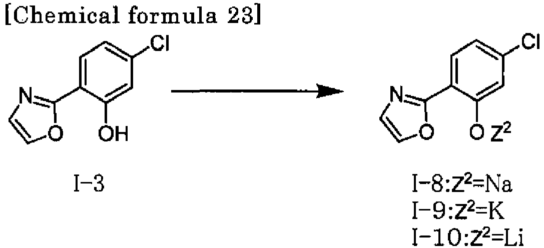

wherein Z² is sodium, potassium or lithium.

### (1) Synthesis of sodium 5-chloro-2-(1,3-oxazole-2-yl)phenolate (I-8)

To 5-chloro-2-(1,3-oxazole-2-yl)phenol(I-3, 20.00 g, 102.2 mmol) was added toluene(200mL) at room temperature. To the reaction mixture was added dropwise methanol solution of 28% sodium methoxide(19.71 g, 102.2 mmol) at 25°C over 45 minutes. The reaction mixture was warmed up to 70°C, followed by cooled to 25°C and stirred for 55 minutes. The resulting solids were collected by filtration to afford sodium 5-chloro-2-(1,3-oxazole-2-yl)phenolate(I-8, 20.67 g, 92.9 %).
Elemental analysis: C 47.14%, H 2.78%, Cl 15.10%, N 6.10%, Na 10.04%
¹H-NMR (DMSO -d6) δppm:6.25(dd, J=8.1Hz, 1.8 Hz, 1H), 6.54(d, J=1.8 Hz, 1H), 7.18(d, J=0.6 Hz, 1H), 7.55(dd, J=21.0 Hz, 8.7Hz, 1H), 7.97(d, J=0.6 Hz, 1H)

### (2) Synthesis of potassium 5-chloro-2-(1,3-oxazole-2-yl)phenolate(I-9)

To 5-chloro-2-(1,3-oxazole-2-yl)phenol(I-3, 20.00 g, 102.2 mmol) was added toluene(100mL) at room temperature. The reaction mixture was warmed up to 75°C. To the reaction mixture was added dropwise ethanol solution of 24% potassium ethoxide(35.77 g, 102.2 mmol) at 70°C over 50 minutes. To the reaction mixture was added toluene(100mL). The reaction mixture was cooled to 25°C and stirred for 60 minutes. The resulting solids were collected by filtration to afford potassium 5-chloro-2-(1,3-oxazole-2-yl)phenolate(I-9, 7.76 g, 26.4 %).
Elemental analysis:C 42.41%, H 2.68%, Cl 13.57%, N 5.54%, K 15.73%
¹H-NMR (DMSO-d6) δppm: 6.16(dd, J=8.4 Hz, 2.4 Hz, 1H), 6.38(d, J=2.1 Hz, 1H), 7.14(d, J=1.2 Hz, 1H), 7.53(dd, J=8.1 Hz, 4.2 Hz, 1H), 7.99(d, J=1.2 Hz, 1H)

### (3) Synthesis of lithium 5-chloro-2-(1,3-oxazole-2-yl)phenolate(I-10)

5-chloro-2-(1,3-oxazole-2-yl)phenol(I-3, 10.00 g, 51.2 mmol) was dissolved in toluene(60mL). To a methanol solution of 1 mol/L lithium methoxide(43.84 g, 53.7 mmol) was added the solution at room temperature over 20 minutes. The reaction mixture was stirred for 1 hour 8 minutes. The mixture was added toluene, then the solvent was removed under reduced pressure repeatedly at 40°C, and the solvent was replaced by toluene. The reaction mixture was stirred for 1 hour 30 minutes. The resulting solids were collected by filtration to afford lithium 5-chloro-2-(1,3-oxazole-2-yl)phenolate(I-10, 5.21 g, 50.3 %).
Elemental analysis: C 52.92%, H 2.59%, Cl 17.71%, N 6.95%, Li 3.36%
¹H-NMR (DMSO-d6) δppm: 5.88(dd, J=8.4 Hz, 2.4 Hz, 1H), 6.20(d, J=2.1 Hz, 1H), 7.07(d, J=0.9 Hz, 1H), 7.39(dd, J=15.9 Hz, 7.5 Hz, 1H), 7.84(d, J=1.2 Hz, 1H)

### [Example 3]

### Synthesis of {5-[4-(4-isopropyloxy phenylsulfonyl)-piperazine-1-yl]-2-(1,3-oxazole-2-yl)-phenoxy}-acetic acid(I-6)

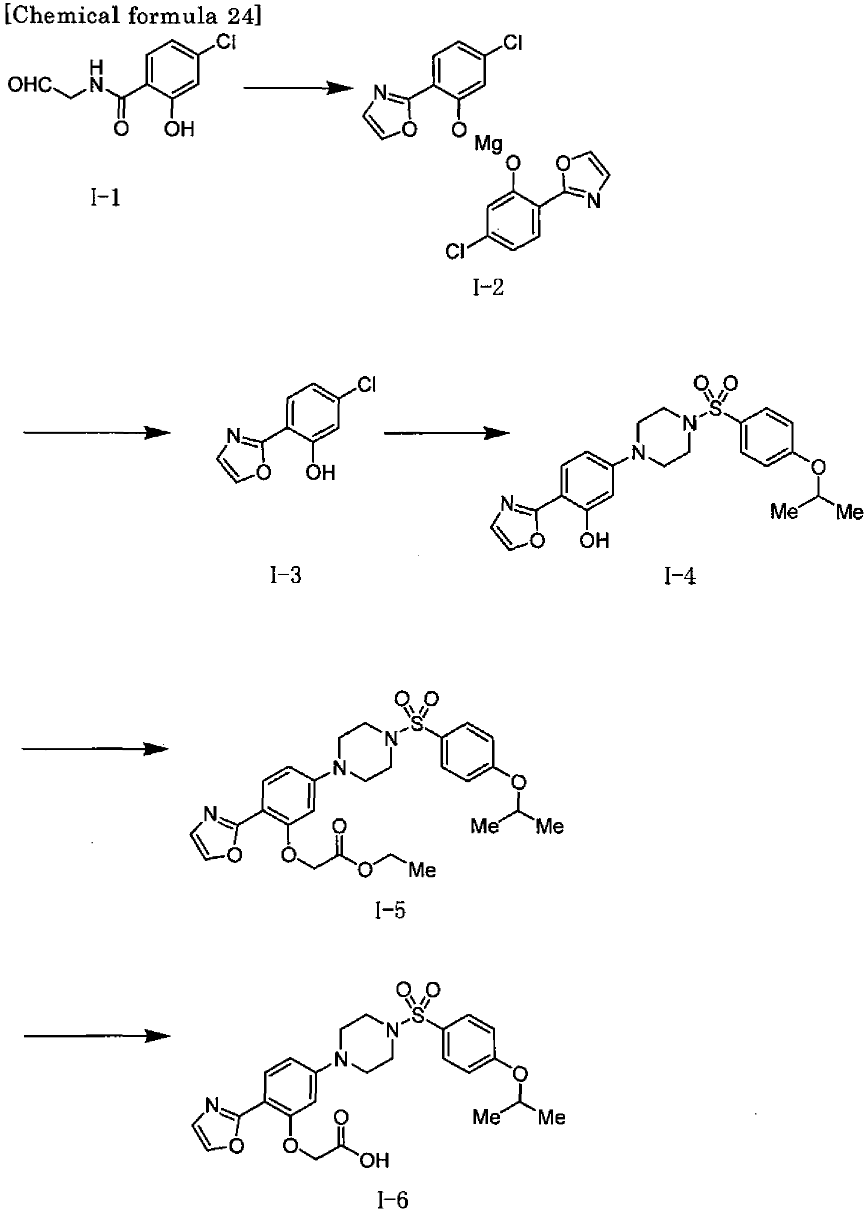

### (1)Synthesis of magnesium bis[5-chloro-2-(1,3-oxazole-2-yl)phenolate](I-2)

Triphenylphosphine (140.96 g, 537.41 mmol) was suspended in acetonitrile(550mL). To the reaction mixture was added chlorine(37.79 g, 532.93 mmol) at 20°C to give an acetonitrile solution of dichlorotriphenylphosphorane(611. 28 g).

4-Chloro-2-hydroxy -N-(2-oxoethyl)benzamide (I-1, 5.97 g, 27.94 mmol) was suspended in acetonitrile(12mL). An acetonitrile solution of dichlorotriphenylphosphorane(42.85 g) was added dropwise to the reaction mixture at 25°C over 1 hour 45 minutes. The reaction mixture was stirred for 55 minutes. Triethylamine(11.31 g, 111.77 mol) was added dropwise to the reaction mixture at 25°C over 2 hours. The reaction mixture was stirred at 25°C for 2 hours 52 minutes. To the reaction mixture was added water(12mL) at 25°C, then warmed up to 60°C. Magnesium chloride hexahydrate(2.84 g, 13.98 mmol) was dissolved in water(6mL). To the reaction mixture was added magnesium chloride solution at 60°C over 1hour. The reaction mixture was cooled to 25°C, then stood overnight. The resulting crystals were collected by filtration to afford magnesium bis[5-chloro-2-(1,3-oxazole-2-yl)phenolate] (I-2, 5.08 g, 96.1%).

### (2)Synthesis of 5-chloro-2-(1,3-oxazole-2-yl)phenol (I-3)

Magnesium bis[5-chloro-2-(1,3-oxazole-2-yl)phenolate](I-2, 15.00 g, 36.28 mmol) was added toluene(90 mL), methanol (15 mL), water(30 mL) and acetic acid(13.07 g, 217.65 mmol) at room temperature. The reaction mixture was warmed up to 60°C, then stirred for 79 minutes. To the reaction mixture were added 30% hydrogen peroxide solution(8.2 mg, 0.07 mmol) and water(15 mL) at 60°C, then stirred for 60 minutes. To the reaction mixture were added sodium hydrogen sulfite(13.7 mg, 0.13 mmol) and water(15mL) at 60°C, then stirred for 30 minutes. The reaction mixture was added 12% brine(30.00 g). The aqueous layer was removed by separating. The obtained organic layer was added toluene, then evaporated under reduced pressure repeatedly at 30°C and replaced with toluene to afford a toluene solution of 5-chloro-2-(1,3-oxazole-2-yl)phenol(I-3) (61.54 g).

### (3)Synthesis of 5-[4-(4-isopropyloxyphenylsulfonyl)-piperazine-1-yl]-2-(1,3-oxazole-2-yl)-phenol (I-4)

4-(4-isopropyloxyphenylsulfonyl)piperazine(11.55 g, 40.62 mmol), sodium tert-pentoxide (11.18 g, 101.51 mmol), tri-tert-butylphosphonium tetraphenylborate (53.1 mg, 0.10 mmol) and palladium acetate(22.8 mg, 0.10 mmol)were suspended in toluene(40 mL). To the reaction mixture was added the toluene solution of 5-chloro-2-(1,3-oxazole-2-yl)phenol(I-3) (28.72 g) at 35°C over 10 minutes. The reaction mixture was warmed up to 110°C, then stirred for 1 hour 30 minutes. The reaction mixture was cooled to 75°C. To the reaction mixture were added tert-butyl alcohol(17mL) and water(13mL) at 75°C, then 16% hydrochloric acid(22.91 g) was added dropwise over 1 hour 14 minutes. The reaction mixture was cooled to 40°C, then added methanol(7mL). The aqueous layer of the reaction mixture was removed at 40°C, then the obtained organic layer was cooled to 25°C. To the reaction mixture was added methanol(12mL). The reaction mixture was stirred for 1 hour, then cooled to 0°C and stirred additionally for 1 hour. The resulting crystals were collected by filtration to afford 5-[4-(4-isopropyloxyphenylsulfonyl)-piperazine-1-yl]-2-(1,3-oxazole-2-yl)-phenol (I-4, 13.15 g, 87.6%).

### (4)Synthesis of {5-[4-(4-isopropyloxyphenylsulfonyl)-piperazine-1-yl]-2-(1,3-oxazole-2-yl)-phenoxy}-carboxylic acid ethyl ester(I-5)

To 5-[4-(4-isopropyloxyphenylsulfonyl)-piperazine-1-yl]-2-(1,3-oxazole-2-yl)-phenol (1-4, 10.00 g, 22.55 mmol) were added potassium iodide(0.75 g, 4.52 mmol), potassium carbonate(4.7 g, 34.01 mmol) and N,N-dimethylformamide (50mL) at room temperature. The reaction mixture was warmed up to 80°C. To the reaction mixture was added dropwise ethyl bromoacetate(5.6 g, 33.53 mmol) at 80°C over 2 hours 50 minutes, then stirred for 1 hour. The reaction mixture was allowed to cool to 25°C. To the reaction mixture was added dropwise acetic acid(4.1 g, 68.28 mmol) at 25°C over 5 minutes, then methanol(50mL) and water(100mL) were added. The reaction mixture was stirred at 25°C for 60 minutes, then stood overnight. The resulting solids were collected by filtration to afford {5-[4-(4-isopropyloxyphenylsulfonyl)-piperazine-1-yl]-2-(1,3-oxazole-2-yl)-phenoxy}-carboxylic acid ethyl ester(I-5, 20.94 g) as moist solid.
Elemental analysis:C 58.84%, H 5.89%, N 7.96%, S 5.76%
¹H-NMR (CDCl₃) δppm: 1.27(t, J= 7.2Hz, 3H), 1.37(d, J=6.3Hz, 6H), 3.13(m, 4H), 3.33(m, 4H), 4.24(q, J=7.2 Hz, 2H), 4.65(m, 1H), 6.38(d, J=2.4 Hz, 1H), 6.56(dd, J=8.7Hz, 2.1Hz), 6.97(d, J=9.0 Hz, 2H), 7.20(d, J=0.9 Hz, 1H), 7.66(d, J=0.6Hz, 1H), 7.68(d, J=9.0Hz, 2H), 7.84(d, J=8.4Hz, 1H)

### (5)Synthesis of 5-[4-(4-isopropyloxyphenylsulfonyl)-piperazine-1-yl]-2-(1,3-oxazole-2-yl)-phenoxy}-acetic acid(I-6)

To the moist solids of {5-[4-(4-isoproppyloxyphenylsulfonyl)-piperazine-1-yl]-2-(1,3-oxazole-2-yl)-phenoxy}-acetic acid ethyl ester(I-5, 20.31 g) were added tetrahydro furan (127mL) and activated charcoal(1.1 g) at room temperature. The reaction mixture was warmed up to 65°C, then stirred for 1 hour. The activated charcoal was removed by filtration at 60°C. To the obtained filtrate was added dropwise 2% sodium hydroxide solution(44.90 g) at 60°C over 20 minutes. The reaction mixture was stirred at 60°C for 1 hour. To the reaction mixture was added dropwise 1% hydrochloric acid solution(65.02 g) at 60°C. The reaction mixture was allowed to cool to 25°C, then stirred for 2 hours. The resulting crystals were collected by filtration, then recrystallized to afford 5-[4-(4-isopropyloxyphenylsulfonyl)-piperazine-1-yl]-2-(1,3-oxazole-2-yl)-phenoxy}-acetic acid(I-6, 9.12 g) as crystal.

### [Example 4]

### Synthesis of 5-chloro-2-[(5RS)-5-chloro-4,5-dihydro-1,3-oxazole-2-yl]phenol hydrochloride (I-11)

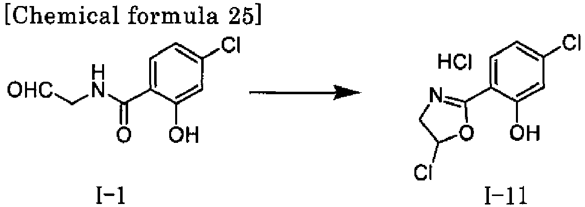

Hexachloroethane(36.56 g, 15.44 mmol) was suspended in acetonitrile(160mL). To the reaction mixture was added triphenylphosphine at 23°C, then the reaction mixture was stirred for 1 hour. To the reaction mixture was added 4-chloro-2-hydroxy -N-(2-oxoethyl)benzamide(I-1, 20.00 g, 93.62 mmol) at 23°C, then the reaction mixture was stirred for 1 hour. The resulting solids were collected by filtration to afford 5-chloro-2-[(5RS)-5-chloro-4,5-dihydro-1,3-oxazole-2-yl]phenol hydrochloride(I-11, 19.0 g).
Elemental analysis:C 40.07%, H 2.93%, N 5.33%, S 39.85%
¹H-NMR (CDCl₃) δppm:4.38(dd, J=16.5 Hz, 2.0 Hz, 1H), 4.54(dd, J=16.5 Hz, 6.5 Hz, 1H), 6.56(dd, J=6.5 Hz, 2.0Hz), 6.91(dd J=8.5 Hz, 1.7Hz), 7.07(d J=1.7Hz), 7.61(d, J=8.5Hz)

Magnesium bis[5-chloro-2-(1,3-oxazole-2-yl)phenolate](I-2) can be synthesized from the operation after adding triethylamine described in the Example 3(1), relative to the above 5-chloro-2-[(5RS)-5-chloro-4,5-dihydro-1,3-oxazole-2-yl]phenol hydrochloride (I-11).

### Measurement of an X-ray powder diffraction pattern

X-ray powder diffraction measurement of the crystal obtained in each example was performed on the following measurement conditions in accordance with the X-ray powder diffraction method described to General Test Procedures of the Japanese pharmacopoeia.

### (Device)

### MiniFlexII(Rigaku)

### (Operation method)

As to each sample, the following measurement condition was adopted.
Measuring method: Reflection method
The kind of light source: Cu bulb
Operating wavelength: CuKα rays
Tube current: 15 mA
Tube voltage: 30 Kv
Sample plate: non-reflecting sample plate, silicon
Scan speed: 5.000°/minute
Scan range4.000∼40.0000°
Sampling range:0.0200°

Crystal can be characterized by the value of the each diffraction angle or surface separation, d sinθ=nλ: n is an integer, d is surface separation(unit: angstrom), θ is diffraction angle(unit: degree)

### Test example

### Test example 1: evaluation of sublimation by measuring TG/DTA

(1) 5-Chloro-2-(1,3-oxazole-2-yl)phenol (I-3, 5 mg) which was obtained from the method described in Patent Document 6 was measured, and an aluminum pan was stuffed with it and measured in the open system. The measurement conditions are as follows.

### (Measurement conditions)

Device: TG/DTA 7200 by SII nano technology
Measurement temperature range: 30°C-200°C
Heating rate:10°C/minute
Purge gas: N₂ 200 mL/minute

Result of TG/DTA analysis is shown in Figure 3. The onset temperature of melting is about 90°C, the weight loss of 0.38% was observed at 40-85°C.

(2) 5-Chloro-2-(1,3-oxazole-2-yl)phenol (I-3, about 10 mg) which was obtained from the method described in Patent Document 6 was measured, and an aluminum pan was stuffed with it and measured in the open system. The measurement conditions are as follows.

### (Measurement conditions)

Device: TG/DTA 7200 by SII nano technology
Measurement temperature range: 40°C
Measurement time: 720 minutes
Purge gas: N₂ 200 mL/minute

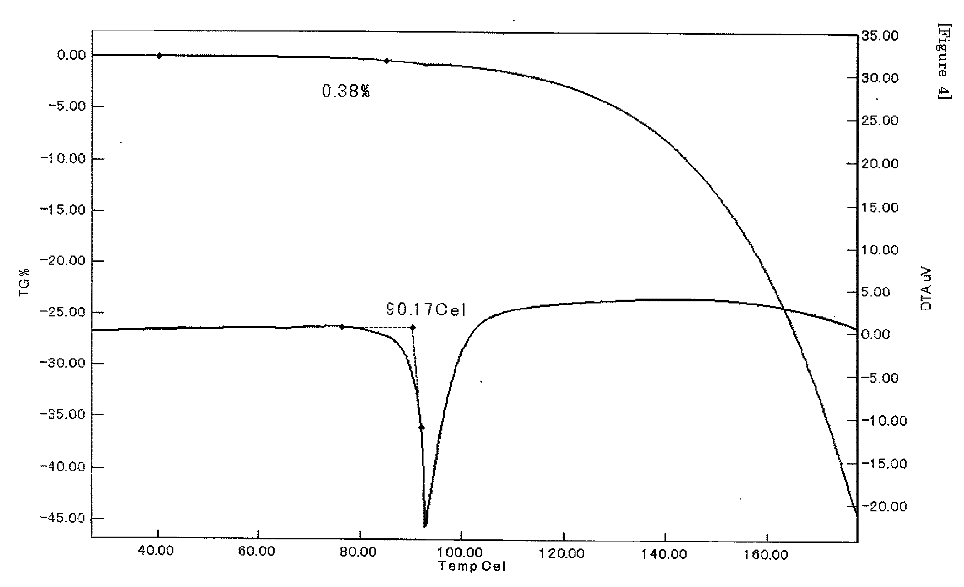

Result of TG/DTA analysis is shown in Figure 4. The sample was solid state under low temperature condition at 40°C, the weight loss of 1.6 % was observed. This result indicated that 5-Chloro-2-(1,3-oxazole-2-yl)phenol (I-3) has sublimation. (3) Magnesium bis[5-chloro-2-(1,3-oxazole-2-yl)phenolate (I-2, 5mg) which was obtained from Example1(1) was measured, and an aluminum pan was stuffed with it and measured in the open system. The measurement conditions are as follows.

### (Measurement conditions)

Device: TG/DTA 7200 by SII nano technology
Measurement temperature program: 30°C-200°C
Rate of temperature increase: 10°C/minute
Purge gas: N₂ 200 mL/minute

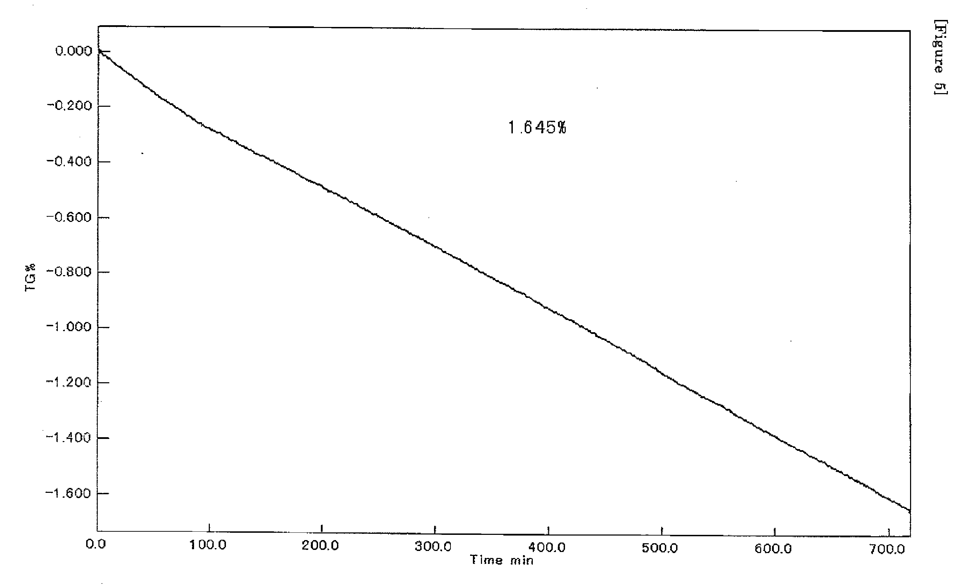

Result of TG/DTA analysis is shown in Figure 5. Under about 30°C-about 180°C, the melting and the weight loss were not observed. This result indicated that magnesium bis[5-chloro-2-(1,3-oxazole-2-yl)phenolate(I-2) doesn't have sublimation.

### Test example 2: evaluation of sublimation

5-Chloro-2-(1,3-oxazole-2-yl)phenol (I-3, 3.01 g, 15.4 mmol) which was obtained from the method described in Patent Document 6 was measured in a roundbottomed flask with condenser tube filled with -15°C cooling liquid, and heated at 40°C for 6 hours under the reduced pressure(16∼18 mmHg). After 6 hours, 0.02 g of colorless solids were adhered to the condenser tube. The colorless solids were measured by ¹H -NMR (DMSO-d6), it was confirmed to be 5-chloro-2-(1,3-oxazole-2-yl)phenol(I-3). This result indicated that 5-chloro-2-(1,3-oxazole-2-yl)phenol(I-3) has sublimation.

### Test example 3: evaluation of hygroscopicity by measuring moisture adsorption-desorption isotherm

Moisture adsorption-desorption isotherm of the compounds specified in following (1) to (4) obtained from corresponding examples were measured by the following conditions.

### (Device)

### IGASorp by Hiden Isochema

### (Operating method)

About 50 mg of the sample was weighted in the mesh stainless-steel sample container, then it was dried at the relative humidity(hereinafter, RH) 0% until the mass change doesn't happen in the device. Temperature and humidity were controlled by the following conditions, and dynamic gravimetric measurement was conducted.
Measurement temperature: 25°C
Measurement humidity range: adsorption 0∼95% RH, desorption 95∼0% RH, 5% RH interval
Measurement condition: Isotherm condition

Herein, isotherm condition means that measurement is move on to next humidity point when the mass change is no longer observed after minimum waiting time or when the equilibrium of mass was not observed until maximum waiting time under constant humidity.

The minimum waiting time for equilibrium determination at each humidity: 10 minutes

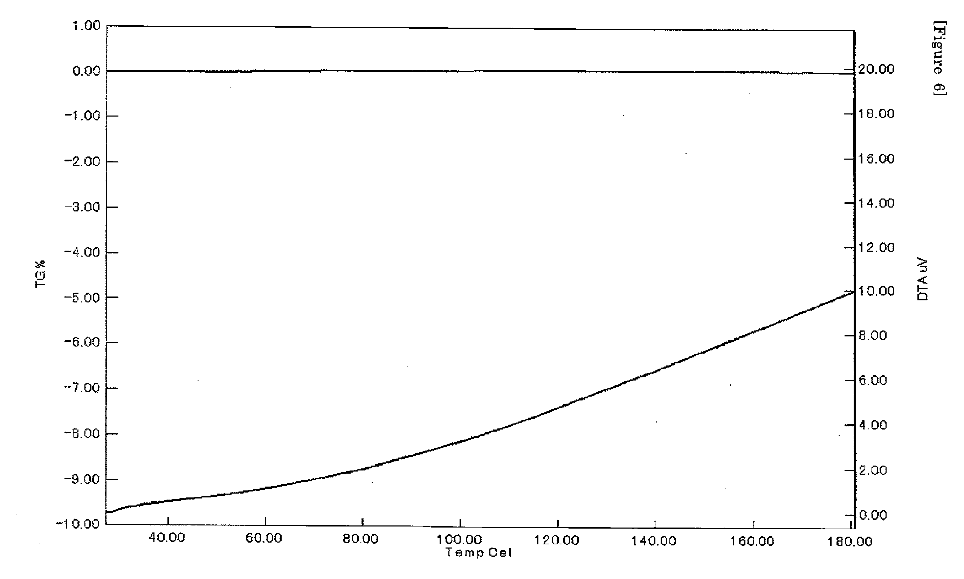

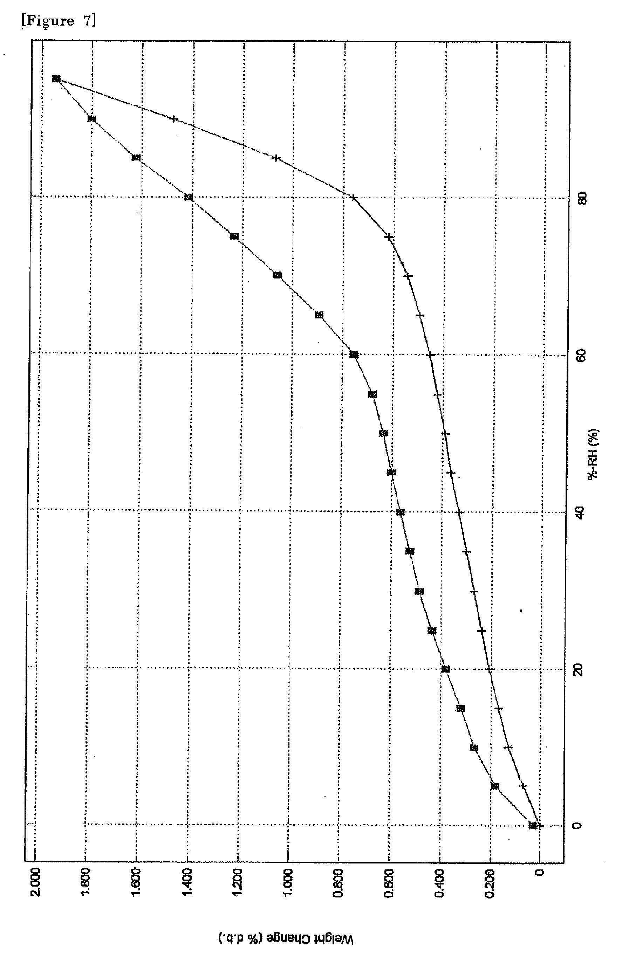

The maximum waiting time for equilibrium determination at each humidity: 2 hours Humidity control: nitrogen and humidification device, 250mL/minutes
(1) Result of the moisture adsorption-desorption isotherm for magnesium bis[5-chloro-2-(1,3-oxazole-2-yl)phenolate] (I-2) which was obtained from Example1(1) is shown in Figure 6. In measuring the moisture adsorption isotherm, in the case of 95% RH, the ratio of mass increase was 2 % relative to the mass at 0% RH. In measuring the moisture desorption isotherm, in the case of 0% RH, the ratio of mass increase was 0%.
(2) Result of the moisture adsorption-desorption isotherm for calcium bis[5-chloro-2-(1,3-oxazole-2-yl)phenolatel (I-7) which was obtained from Example1(2) is shown in Figure 7. In measuring the moisture adsorption isotherm, in the case of 95% RH, the ratio of mass increase was 3 % relative to the mass at 0% RH. In measuring the moisture desorption isotherm, in the case of 0% RH, the ratio of mass increase was 0%.

### [Industrial applicability]

The present invention provides the salt and the crystal thereof of 2-(1,3-oxazole-2-yl)phenol derivative without hygroscopicity and sublimation, which are efficient intermediate in the process of producing piperazine derivative, and the process of producing the salt as well as the process of producing N-phenyl-N'-phenylsulfonyl piperazine derivative through the salt.

### [Brief description of Drawings]

[Figure 1] Figure 1 shows an X-ray powder diffraction pattern and its peaks of the mixture of polymorphism of the crystal of the salt of the compound (I-2) obtained from Example 1(1). The vertical axis indicates intensity and the horizontal axis indicates diffraction angle [2θ, unit: °].
[Figure 2] Figure 2 shows an X-ray powder diffraction pattern and its peaks of single substance of polymorphism of the crystal of the salt of the compound (I-2) obtained from Example 1(1). The vertical axis indicates intensity and the horizontal axis indicates diffraction angle [2θ, unit: °].
[Figure 3] Figure 3 shows an X-ray powder diffraction pattern and its peaks of single substance of polymorphism of the crystal of the salt of the compound (1-2) obtained from Example 1(1). The vertical axis indicates intensity and the horizontal axis indicates diffraction angle[2θ, unit: °].
[Figure 4] Figure 4 shows result of TG/DTA curve of the compound (I-3) obtained from the method described in Patent document 6 in the rate of temperature increase 10°C/minute. The upper curve indicates TG curve and the bottom curve indicates DTA curve. The vertical axis indicates the change of mass and heat quantity [TG, unit:%, and DTA, unit: uV], and the horizontal axis indicates temperature [TempCel, unit: °C].
[Figure 5] Figure 5 shows result of TG curve of the compound (1-3) obtained from the method described in Patent document 6 at 40°C. The vertical axis indicates mass[TG, unit: %], and the horizontal axis indicates time [Time min, unit: minutes].
[Figure 6] Figure 6 shows result of TG/DTA curve of the compound (I-2) obtained from Example 1(1) in the rate of temperature increase 10°C/minute. The upper curve indicates TG curve and the bottom curve indicates DTA curve. The vertical axis indicates the change of mass and heat quantity [TG, unit:%, and DTA, unit: uV], and the horizontal axis indicates temperature [TempCel, unit: °C].
[Figure 7] Figure 7 shows result of the moisture adsorption-desorption isotherm of the salt for the compound (I-2) which was obtained from Example1(1). The vertical axis indicates the ratio of mass increase relative to the mass at 0% RH[Weight Change, unit: %], and the horizontal axis indicates relative humidity[RH, unit: %]. The curve of plotting + indicates the moisture adsorption isotherm, and the curve of plotting ■ indicates the moisture desorption isotherm.

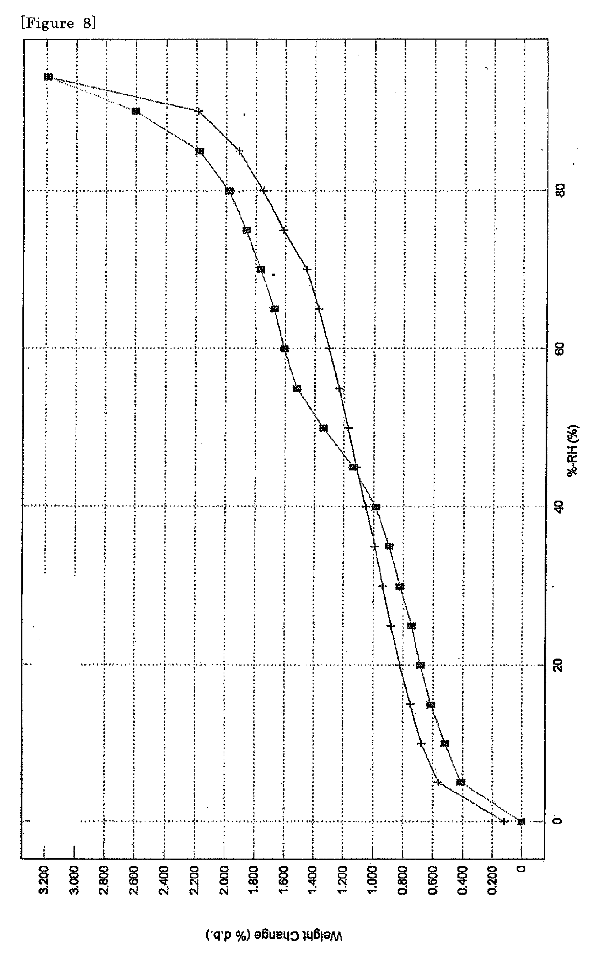

[Figure 8] Figure 8 shows result of the moisture adsorption-desorption isotherm of the salt for the compound (I-7) which was obtained from Example 1(2). The vertical axis indicates the ratio of mass increase relative to the mass at 0% RH[Weight Change, unit: %], and the horizontal axis indicates relative humidity[RH, unit: %]. The curve of plotting + indicates the moisture adsorption isotherm, and the curve of plotting ■ indicates the moisture desorption isotherm.

## Claims

1. A salt of the compound represented by Formula (I): 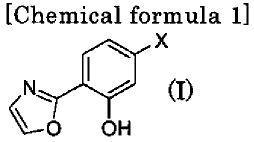 wherein X is halogen.

2. The salt according to claim 1, wherein said salt is magnesium salt or alkaline earth metal salt.

3. The salt according to claim 1 or 2, wherein said salt is magnesium salt or calcium salt.

4. The salt according to any one of claims 1 to 3, wherein X is chlorine.

5. A crystal of magnesium salt or alkaline earth metal salt of the compound represented by Formula (I): 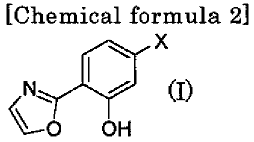 wherein X is halogen.

6. The crystal according to claim 5, wherein said crystal is magnesium salt of the compound represented by Formula (I), when X is chlorine.

7. A process for producing magnesium salt or alkaline earth metal salt of the compound represented by Formula (I): 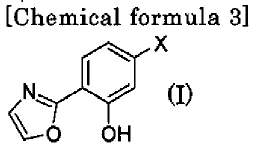 wherein X is halogen;
**characterized by** treating the compound represented by Formula (I) with magnesium halide, magnesium hydride, magnesium hydroxide, magnesium oxo acid salt, magnesium organic acid salt, alkaline earth metal halide, alkaline earth metal hydride, alkaline earth metal hydroxide, alkaline earth metal oxo acid salt or alkaline earth metal organic acid salt.

8. The process according to claim 7, comprising the step of producing the compound represented by Formula (I): 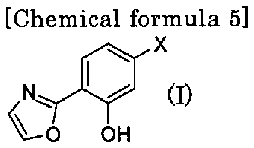 wherein X is halogen;
by cyclization of the compound represented by Formula (II): 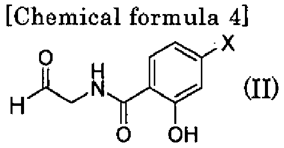 wherein X is as defined above; or a salt thereof.

9. The process according to claim 8, wherein the compound represented by Formula (II) or a salt thereof is cyclized in the presence of triphenylphosphine, a halogenating agent and a base.

10. A process for producing the compound represented by Formula (VIII): 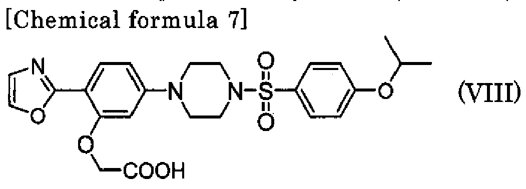 or a salt thereof, comprising the process through magnesium salt or alkaline earth metal salt of the compound represented by Formula (I): 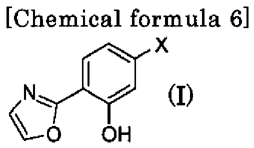 wherein X is halogen.
